# EUROPÄISCHE PATENTSCHRIFT

(11) **EP 2 494 928 B1**
(45) Veröffentlichungstag und Bekanntmachung des Hinweises auf die Patenterteilung: **17.01.2018**
(21) Anmeldenummer: 11156583.4
(22) Anmeldetag: 02.03.2011
(51) Int. Cl.: A61B 17/00, G06F 3/01, A61B 34/20, A61B 90/00

(54) **Bedieneinrichtung für eine technische Vorrichtung, insbesondere eine medizinische Vorrichtung**
Operating device for a technical device, in particular a medical device
Dispositif de commande pour un dispositif technique, notamment un dispositif médical

(43) Veröffentlichungstag der Anmeldung: 05.09.2012
(73) Patentinhaber: Siemens Aktiengesellschaft, 80333 München (DE)
(72) Erfinder: Feiten, Wendelin, 85579 Neubiberg (DE); Kagermeier, Robert, 90427 Nürnberg (DE); Prummer, Simone, 91077 Neunkirchen am Brand (DE)

(56) Entgegenhaltungen:
- WO-A2-2004/001569
- DE-A1-102009 037 316
- US-A- 5 526 022
- US-A1- 2002 012 014
- US-A1- 2005 264 527
- US-A1- 2009 096 746
- US-A1- 2009 326 406
- US-A1- 2009 327 171
- US-B1- 6 244 873

## Beschreibung

Die Erfindung betrifft eine Bedieneinrichtung für eine technische Vorrichtung, insbesondere eine medizinische Vorrichtung.

Bei der Bedienung von technischen Vorrichtungen ist es oftmals wünschenswert, dass berührungslos Befehle für den Betrieb der technischen Vorrichtung generiert werden. In bestimmten Szenarien werden die Hände des Benutzers hauptsächlich für andere manuelle Arbeiten als für die Eingabe von Befehlen an einer technischen Vorrichtung benötigt, so dass es für ihn schwierig ist, neben der Durchführung dieser manuellen Arbeiten gleichzeitig auch nach die technische Vorrichtung zu bedienen. Diese Problematik tritt insbesondere im Bereich der Medizintechnik auf, z.B. bei der Durchführung von Operationen. Dabei ist zusätzlich sicherzustellen, dass die zu bedienende Vorrichtung möglichst nicht berührt wird, damit der Bediener steril bleibt.

Aus dem Stand der Technik sind Systeme bekannt, die steuernde Gesten eines Benutzers mit optischen Sensoren erkennen, wobei diese Gestenerkennung durch Verwerdung weiterer Informationsquellen unterstützt und verbessert wird. Solche Systeme sind jedoch nicht genau genug, um hierüber eine Einsteuerung einer technischen Vorrichtung im Bereich von wenigen Grad oder Zentimetern zu erreichen. Aus der Druckschrift T. Scott Saponas et al., »Demonstrating the Feasibility of Using Forearm Electromyography for Muscle-Computer Interfaces«, CHI 2008, 5.- 10. April, 2008, Florenz, Italien, ist ferner bekannt, durch elektromyographische Ableitung der Aktivierungspotentiale von Muskeln berührungslose digitale Eingaben zu machen. Ferner sind im Stand der Technik Systeme beschrieben, mit denen über an eine Person angebrachte Beschleunigungssensoren Lageänderungen und Bewegungen des Körpers der Person erfasst werden.

Die Druckschrift US 2002/0012014 A1 offenbart die Erfassung der Lage und Bewegung der Hand eines Benutzers über eine Vielzahl von Sensoren. Dabei wird sowohl die Lage des Handrückens als auch die Lage der einzelnen Finger der Hand sensiert. Basierend auf den Sensorsignalen werden Kommandos generiert.

Aufgabe der Erfindung ist es, eine Bedieneinrichtung für eine technische Vorrichtung zu schaffen, welche eine zuverlässige Erkennung von mittels einer Hand des Benutzers spezifizierten Befehlen für den Betrieb der technischen Vorrichtung ermöglicht.

Diese Aufgabe wird durch die Bedieneinrichtung gemäß Patentanspruch 1 gelöst. Weiterbildungen der Erfindung sind in den abhängigen Ansprüchen definiert.

Über die erfindungsgemäße Bedieneinrichtung kann ein Benutzer mittels seiner Hand berührungslos Befehle für den Betrieb der technischen Vorrichtung generieren. Unter berührungsloser Eingabe ist dabei die Eingabe von Befehlen mit der Hand des Benutzers ohne Kontakt zu der technischen Vorrichtung zu verstehen. Die erfindungsgemäße Bedieneinrichtung umfasst eine erste Sensoreinrichtung zum Erfassen der Lage der Hand des Benutzers im Raum, wodurch erste Sensorsignale generiert werden. Dabei wird insbesondere die dreidimensionale Lage der Hand im Raum erfasst. Ferner umfasst die Bedieneinrichtung eine unabhängig von der ersten Sensoreinrichtung sensierende zweite Sensoreinrichtung zum Erfassen der Bewegung zumindest eines Teils der Finger der Hand des Benutzers, wodurch zweite Sensorsignale generiert werden. Es werden somit zwei unabhängige, im Rahmen der Sensierung nicht zusammenarbeitende Sensoreinrichtungen verwendet, um zum einen die Bewegung der gesamten Hand des Benutzers und zum anderen die Relativbewegung der einzelnen Finger in Bezug auf die Hand des Benutzers zu erfassen. Dabei umfasst die Bedieneinrichtung eine Verarbeitungseinheit, welche die ersten und zweiten Sensorsignale der ersten und zweiten Sensoreinrichtung derart verarbeitet, dass ermittelt wird, welches oder welche vorbestimmten, jeweilige Befehle eines Benutzer repräsentierende Bewegungsmuster die Sensorsignale darstellen. Der oder die jeweiligen Befehle können dann von der technischen Vorrichtung zu deren Ausführung weiterverarbeitet werden.

Die erfindungsgemäße Bedieneinrichtung zeichnet sich durch eine geeignete Kombination von zwei unabhängigen Sensoreinrichtungen aus, wobei durch eine Auswertung beider Sensorsignale mit hoher Genauigkeit erkannt werden, welche Befehle der Benutzer durch die freie Bewegung seiner Hand bzw. seiner Finger spezifiziert.

Erfindungsgemäß ist die Verarbeitungseinheit der Bedieneinrichtung derart ausgestaltet, dass aus den ersten Sensorsignalen Bewegungen der Hand des Benutzers ermittelbar sind, welche vorbestimmte, durch die technische Vorrichtung durchzuführende Bewegungen repräsentieren. Beispielsweise kann der Benutzer durch die Bewegung seiner Hand eine erwünschte Bewegung eines Roboters bzw. einer medizinischen Vorrichtung nachfahren, die anschließend durch den Roboter bzw. die medizinische Vorrichtung ausgeführt wird. Ist die medizinische Vorrichtung beispielsweise eine Röntgeneinrichtung mit einem sogenannten C-Bogen, so kann der Benutzer im Rahmen des Röntgens eines Patienten eine erwünschte Bewegung des C-Bogens durch die Bewegung seiner Hand im Raum spezifizieren.

In der erfindungsgemäßen Bedieneinrichtung ist die Verarbeitungseinheit ferner derart ausgestaltet, dass aus den zweiten Sensorsignalen virtuelle, über zumindest einen Teil der Finger der Händ des Benutzers durchgeführte Tastenbetätigungen einer virtuellen Eingabeeinrichtung ermittelbar sind. Die virtuellen Tastenbetätigungen stellen vorzugsweise die Betätigung einer Eingabeeinrichtung in der Form einer virtuellen Tastatur oder einer virtuellen Maus dar.

Erfindungsgemäß ist die Verarbeitungseinheit der erfindungsgemäßen Bedieneinrichtung derart ausgestaltet, dass im Rahmen der Ermittlung der oben beschriebenen virtuellen Tastenbetätigungen ferner bestimmt wird, welche Taste der virtuellen Eingabeeinrichtung betätigt
wird, wobei hierzu neben den zweiten Sensorsignalen die ersten Sensorsignale ausgewertet werden, um über die ersten Einsorsignale die Position der Hand des Benutzers in Bezug auf die virtuelle Eingabeeinrichtung während einer virtuellen Tastenbetätigung zu ermitteln. Auf diese Weise können geeignete alphanumerische Eingaben einfach und effizient erfasst werden. Beispielsweise kann der Benutzer durch Tippen mit seinen Fingern in der Luft alphanumerische Eingaben machen, die dann von der technischen Vorrichtung geeignet weiterverarbeitet werden.

In einer besonders bevorzugten Ausführungsform der erfindungsgemäßen Bedieneinrichtung umfasst die erste Sensoreinrichtung einen Beschleunigungssensor, welcher insbesondere einen oder mehrere Drehratensensoren zur Detektion von dreidimensionalen Rotationsgeschwindigkeiten und einen oder mehrere Linearbeschleunigungs-Sensoren zur Detektion von dreidimensionalen Linearbeschleunigungen umfasst. Solche Sensoren sind an sich aus dem Stand der Technik bekannt und in einer besonders bevorzugten Ausführungsform wird der von der japanischen Firma ZMP entwickelte Beschleunigungssensor »e-nuvo IMU-Z« verwendet. In einer weiteren Ausführungsform umfasst die erste Sensoreinrichtung ferner einen Magnetfeldsensor zum Erfassen des Erdmagnetfeldes, mit dem die Lageerkennung der Hand des Benutzers weiter verbessert werden kann. Der oben genannte Sensor der Firma ZMP enthält neben den Drehraten- und Linearbeschleunigungs-Sensoren auch einen solchen Magnetfeldsensor.

Um die Bewegung der Finger bzw. der Hand durch die erste Sensoreinrichtung möglichst wenig einzuschränken, ist in einer weiteren Ausgestaltung der Erfindung die erste Sensoreinrichtung auf dem Handrücken der Hand des Benutzers befestigbar.

In einer weiteren, besonders bevorzugten Ausführungsform der erfindungsgemäßen Bedieneinrichtung umfasst die zweite Sensoreinrichtung eine elektromyographische Sensoreinrichtung zur Erfassung der elektrischen Muskelaktivität bei der Bewegung der Finger der Hand des Benutzers. Solche elektromyographischen Sensoreinrichtungen sind an sich bekannt und in der oben genannten Druckschrift T. Scott Saponas et al. beschrieben. Vorzugsweise umfasst die zweite Sensoreinrichtung dabei ein am Unterarm des Benutzers befestigbares Elektrodenband, in dem eine Mehrzahl von Elektroden zur Erfassung der elektrischen Muskelaktivität. enthalten ist. Die Elektroden haben dabei Kontakt zur Hauptoberfläche, um die bei der Muskelaktivität erzeugten elektrischen Impulse zu sensieren. Das Elektrodenband kann beispielsweise über einen Klettverschluss fest mit dem Unterarm des Benutzers verbunden werden.

Die Elektroden in dem Elektrodenband sind in einer bevorzugten Ausführungsform in Elektrodenpaaren von jeweils zwei sich bei Befestigung des Elektrodebands am Unterarm in Längsrichtung des Unterarms erstreckenden Elektroden angeordnet. Vorzugsweise sind die Elektrodenpaare dabei mit einem Signalbus zum Übermitteln der über die jeweiligen Elektrodenpaare erfassten Muskelaktivität an eine Auswerteeinheit, insbesondere einen Mikrocontroller, verbindbar. Hierdurch kann eine einfache und schnelle Auswertung der durch die Elektroden erfassten Muskelaktivität erreicht werden. Die Auswerteeinheit und insbesondere der Mikrocontroller können gegebenenfalls am Elektrodenband angeordnet sein bzw. in diesem integriert sein.

Um ausreichend gute Signale zu generieren, ist für jedes Elektrodenpaar vorzugsweise ein Eingangsverstärker zur Verstärkung der vom jeweiligen Elektrodenpaar erfassten Signale vorgesehen. Um die von den Elektrodenpaaren stammenden Signale auf den Signalbus zu legen, wird in einer bevorzugten Ausführungsform ein Schieberegister verwendet, welches aufeinander folgend die Signale der Elektrodenpaare von einem Elektrodenpaar zum nächsten auf den Signalbus legt. In der detaillierten Beschreibung wird eine konkrete Realisierung eines solchen Schieberegisters beschrieben.

In einer weiteren, besonders bevorzugten Ausführungsform umfasst die Bedieneinrichtung ferner eine Ausgabeeinrichtung, welche derart mit der Verarbeitungseinheit wechselwirkt, dass sie im Betrieb der Bedienungseinrichtung dem Benutzer die von ihm generierten Befehle bestätigt. Die Ausgabeeinrichtung kann dabei eine optische Ausgabeeinrichtung, insbesondere einen Bildschirm, und/oder eine akustische Ausgabeeinrichtung und/oder eine haptische Ausgabeeinrichtung umfassen. Beispielsweise kann bei der Spezifikation einer durch die technische Vorrichtung durchzuführenden Bewegung mittels der Hand des Benutzers diese Bewegung auf einem Bildschirm angezeigt werden. Insbesondere erfolgt die Anzeige dabei derart, dass ein zweidimensionales oder dreidimensionales Modell der technischen Vorrichtung auf dem Bildschirm wiedergegeben wird und die Bewegung, die von der Bedieneinrichtung basierend auf der Handbewegung des Benutzers ermittelt wurde, durch das Modell der technischen Vorrichtung auf dem Bildschirm durchgeführt wird. Stimmt die Bewegung des Modells dabei mit der tatsächlich vom Benutzer gewünschten Bewegung überein, kann der Benutzer dies durch einen entsprechende Befehl bestätigen, wobei der Befehl wiederum über die Bedieneinrichtung berührungslos eingegeben werden kann, z.B. durch die oben beschriebene Betätigung einer virtuellen Taste.

In einer weiteren Ausgestaltung der erfindungsgemäßen Bedieneinrichtung sind als haptische Ausgabeeinrichtung eine ober mehrere Vibrationsgeber vorgesehen, welche vorzugsweise in der ersten und/oder zweiten Sensoreinrichtung integriert sind. Auf diese Weise kann dem Benutzer einfach und intuitiv rückgemeldet werden, wenn ein entsprechender Befehl durch die Bedieneinrichtung aufgrund der Bewegung der Hand bzw. der Finger des Benutzers erfasst wurde.

In einer weiteren Ausführungsfarm ist die erfindungsgemäße Bedieneinrichtung derart ausgestaltet, dass in deren Betrieb die ersten und/oder zweiten Sensorsignale drahtlos, insbesondere über Bluetooth und/oder WLAN, und/oder drahtgebunden an die Verarbeitungseinheit übermittelt werden. Die drahtlose Übermittlung hat dabei den Vorteil, dass keine Kabel an den entsprechenden Sensoreinrichtungen angebracht werden müssen, welche unter Umständen die Bewegung der Hand des Benutzers stören könnten.

Die Erfindung betrifft darüber hinaus eine technische Vorrichtung, insbesondere eine medizinische Vorrichtung, umfassend die oben beschriebnen Bedieneinrichtung, wobei der oder die jeweiligen Befehle gemäß den von der Bedieneinrichtung ermittelten Bewegungsmustern von der technischen Vorrichtung in deren Betrieb ausgeführt werden. Die technische Vorrichtung ist vorzugsweise eine bildgebende medizinische Vorrichtung, insbesondere eine Röntgeneinrichtung und besonders bevorzugt ein C-Bogen.

Ausführungsbeispiele der Erfindung werden nachfolgend anhand der beigefügten Figuren detailliert beschrieben.

Es zeigen:
- Fig. 1: eine Ausführungsform einer erfindungsgemäßen Bedieneinrichtung, deren Sensoreinrichtungen an der Hand bzw. am Unterarm des Benutzers angebracht sind;
- Fig. 2A und 2B: schematische Darstellungen, welche das Auslesen von Signalen aus dem in Fig. 1 gezeigten Elektrodenband gemäß einer Ausführungsform der Erfindung verdeutlichen; und
- Fig. 3: eine andere Ansicht des in Fig. 1 gezeigten Elektrodenbands.

Die nachfolgend beschriebene Ausführungsform der Erfindung dient zur berührungslosen Bedienung einer medizinischen Vorrichtung wie z.B. einer Röntgeneinrichtung in der Form eines sogenannten C-Bogens. Nichtsdestotrotz kann die beschriebene Ausführungsform auch zur Bedienung beliebiger anderer technischer Vorrichtungen in der Form von Maschinen bzw. Anlagen eingesetzt werden. Unter berührungsloser Bedienung ist dabei eine Bedienung zu verstehen, bei der ein Benutzer ohne Kontaktierung der zu bedienenden Vorrichtung Befehle spezifizieren kann, welche durch die Vorrichtung auszuführen sind.

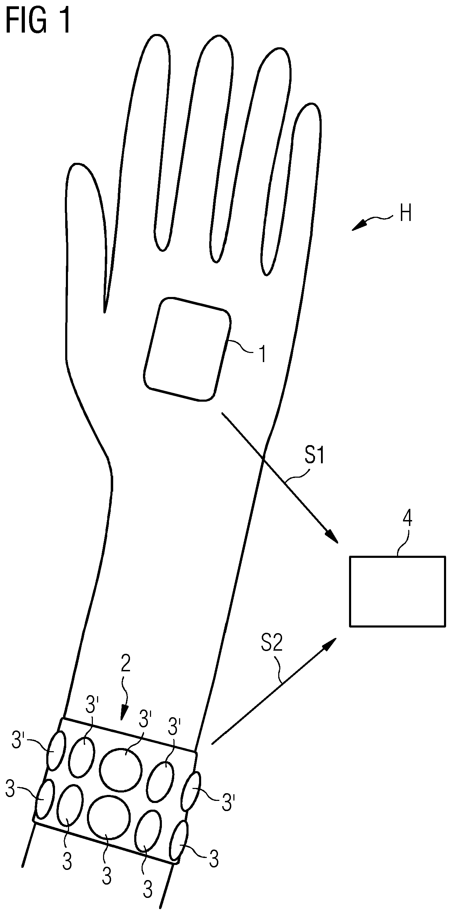

Um eine derartige berührungslose Bedienung zu erreichen, werden gemäß der Ausführungsform der Fig. 1 zwei Sensoreinrichtungen 1 und 2 verwendet. Die Sensoreinrichtung 1 stellt dabei einen Beschleunigungssensor dar, der zum einen einen Sensor zur Erfassung von dreidimensionalen Linearbeschleunigungen und zum anderen einen Gyrosensor zur dreidimensionalen Erfassung von Rotationsbeschleunigungen und damit Winkelgeschwindigkeiten umfasst. Darüber hinaus beinhaltet die Sensoreinrichtung 1 einen Magnetfeldsensor zur dreidimensionalen Erfassung des Erdmagnetfeldes. Der Sensor 1 ist auf dem Handrücken der Hand H eines Benutzers angeordnet. Mit dieser Hand beabsichtigt der Benutzer entsprechende Befehle für die medizinische Vorrichtung zu spezifizieren. Der Aufbau des Sensors 1 ist an sich aus dem Stand der Technik bekannt. Insbesondere sind auch die entsprechenden Sensoren in der Form von Linearbeschleunigungs-Sensoren, Gyrosensoren und Magnetfeldsensoren bekannt. In einer besonders bevorzugten Ausführungsform wird als Sensor 1 der bereits zuvor erwähnte Sensor mit dem Namen »e-nuvo IMU-Z« verwendet. Über den Beschleunigungssensor 1 kann hochgenau die Lage der Hand des Benutzers dreidimensional im Raum erfasst werden. Die entsprechenden Sensorsignale S1 des Sensors 1 werden in der Ausführungsform der Fig. 1 drahtlos an eine Auswerteeinheit 4 übermittelt, welche lediglich schematisch angedeutet ist und insbesondere in der Form einer entsprechenden Recheneinheit (z.B. eines Computers) realisiert ist.

Neben der Sensoreinrichtung 1 wird in der Ausführungsform der Fig. 1 ferner die Sensoreinrichtung 2 eingesetzt, bei der es sich um eine elektromyographische Sensoreinrichtung handelt, mit der entsprechende Muskelaktivitäten am Unterarm des Benutzers erfasst werden. Der elektromyographische Sensor ist dabei als ein Elektrodenband ausgestaltet, in dem eine Vielzahl von Elektroden 3 bzw. 3' integriert sind. Die einzelnen Elektroden im Elektrodenband kontaktieren dabei die Haut des Unterarms, um hierdurch die Muskelaktivitäten im Unterarm elektrisch zu sensieren. Im Unterschied zum Sensor 1 können mit dem elektromyographischen Sensor 2 die Betätigungen der einzelnen Finger des Benutzers basierend auf den erfassten elektrischen Muskelaktivitäten im Unterarm über die Auswertung der Sensorsignale in der Auswerteeinheit 4 bestimmt werden. Die Erfassung von Fingerbewegungen basierend auf einem elektromyographischen Sensor ist dabei an sich aus dem Stand der Technik bekannt und wird beispielsweise in der Druckschrift T. Scott Saponas et al. näher erläutert. Es wird deshalb auf eine detaillierte Beschreibung der Wandlung der erfassten elektromyographischen Signale in Fingerbewegungen verzichtet.

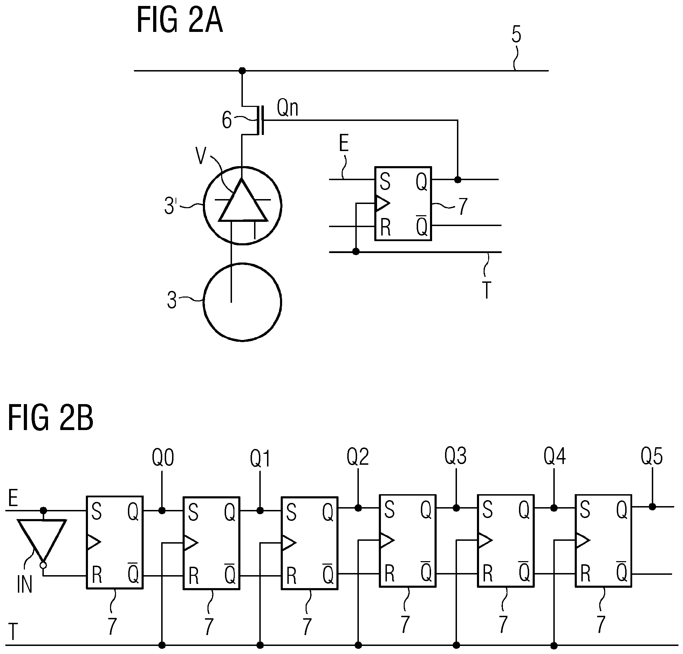

Das in der Fig. 1 dargestellte Elektrodenband umfasst eine obere Reihe mit den Elektronen 3' und eine untere Reihe mit den Elektroden 3, wobei jeweils eine Elektrode der oberen Reihe in Längsrichtung des Unterarms benachbart zu einer Elektroden der unteren Reihe angeordnet ist. Hieraus wird eine Vielzahl von Elektrodenpaaren gebildet. Die Signale jedes Elektrodenpaars, welche sich aus einer entsprechenden Muskelaktivität des unter dem Elektrodenpaar liegenden Muskels ergeben, werden über eine geeignete Schaltanordnung erfasst, welche weiter unten in Bezug auf Fig. 2A und 2B erläutert wird.

Die entsprechend erfassten Elektrodensignale der Sensoreinrichtung 2, welche in Fig. 1 mit S2 bezeichnet sind, werden wiederum in geeigneter Weise drahtlos an die Verarbeitungseinheit 4 übermittelt. Diese Verarbeitungseinheit verarbeitet die Sensorsignale der Sensoren S1 und S2 derart, dass hieraus vorbestimmte Bewegungsmuster abgeleitet werden, welche entsprechende, durch die Bewegung der Hand bzw. der Finger des Benutzers spezifizierte Befehle für die medizinische Vorrichtung repräsentieren. In einer besonders bevorzugten Ausführungsform umfassen die Befehle dabei Bewegungsmuster, mit denen eine erwünschte Bewegung der medizinischen Vorrichtung über die Hand des Benutzers nachgefahren wird. Somit wird die Lageänderung, welche sich aus den Sensorsignalen S1 ergibt, in eine entsprechende Bewegung umgesetzt, welche anschließend von der medizinischen Vorrichtung durchgeführt wird. Insbesondere kann durch die Bewegung der Hand angezeigt werden, wie sich ein C-Bogen einer Röntgeneinrichtung im Rahmen der Untersuchung eines Patienten bewegen soll. Dabei ist die Auswerteeinheit 4 mit einem Bildschirm gekoppelt, auf dem der Benutzer eine Rückmeldung zu der von ihm ausgeführten Bewegung und der damit verknüpften Bewegung der Vorrichtung bekommt, wie weiter unten noch näher erläutert wird.

Im Unterschied hierzu werden aus dem Sensorsignal S2 entsprechende Tastenbetätigungen einer gedachten Tastatur erfasst, welche der Benutzer mittels seiner Hand H in der Luft durchführt. Ein Benutzer kann somit durch Betätigen seiner Finger analog wie bei einer realen Tastatur alphanumerische Befehle eingeben. Dabei wird wiederum der oben erwähnte Bildschirm verwendet, über den der Benutzer eine Rückkopplung zu den von ihm eingegebenen Befehlen bekommt. Insbesondere wird auf dem Bildschirm die über die Finger betätigbare Tastatur wiedergegeben, und ein Benutzer erkennt dann auf dem Bildschirm, wo er sich oberhalb der Tastatur befindet und welche Tasten er bei der Bewegung seiner Finger in der Luft bestätigt. Dabei werden im Rahmen der Tastenbetätigung auch die Sensorsignale S1 des Sensors 1 ausgewertet, um hierdurch Lageänderungen der Hand oberhalb der virtuellen Tastatur festzustellen. Je nach Anwendungsfall können auch andere Betätigungen durch die Bedieneinrichtung der Fig. 1 erfasst werden. Insbesondere besteht auch die Möglichkeit, dass die Bewegung bzw. die Tastenbetätigung einer (virtuellen) Maus durch den Benutzer berührungslos in der Luft durchgeführt wird, wobei die entsprechende Bewegung bzw. Betätigung auf dem bereits oben erwähnten Bildschirm z.B. in der Form einer Cursorbewegung wiedergegeben wird.

Fig. 2A zeigt eines der Elektrodenpaare aus Elektroden 3 und 3' der elektromyographischen Sensoreinrichtung 2 der Fig. 1. Nachfolgend wird erläutert, wie die Elektrodensignale gemäß der hier beschriebenen Ausführungsform ausgelesen werden. Die Elektroden erfassen dabei eine Potentialdifferenz, welche sich durch eine elektrische Muskelaktivität, hervorgerufen durch einen elektrischen Nervenimpuls, ergibt. Diese Potentialdifferenz wird durch einen entsprechenden Eingangsverstärker V verstärkt, wobei jedem Elektrodenpaar in der Sensorenrichtung 2 ein solcher Eingangsverstärker zugeordnet ist. Über einen Transistor 6, der ebenfalls für jedes Elektrodenpaar vorgesehen ist, gelangt das Signal auf einen Signalbus 5, auf den alle Elektrodenpaare des Elektrodenbands zugreifen können. Über ein D-Flip-Flop 7 erfolgt die Steuerung des Transistors, d.h. das Schalten des Transistors, um das Signal des Eingangsverstärkers 7 auf den Signalbus 5 zu geben. Das auf den Signalbus gegebene Signal ist dabei mit Qn bezeichnet.

Das D-Flip-Flop 7 ist Teil eines Schieberegisters, welches beispielhaft für sechs Elektrodenpaare in Fig. 2B gezeigt ist. Der Aufbau dieses Schieberegisters ist an sich bekannt und wird deshalb nicht detailliert erläutert. Die einzelnen Flip-Flops verfügen über einen Set-Eingang S und ein Reset-Eingang R sowie eine Ausgang Q und einen invertierenden Ausgang *Q̅*. Über eine Eingangsleitung E wird ein Signal in der Form einer Bitfolge eingegeben. Dieses Signal wird sowohl an den Set-Eingang S sowie über einen Inverter IN an den Reset-Eingang R geleitet. Darüber hinaus ist eine Taktleitung T vorgegehen, mit der das Schieben der Bits im Schieberegister gemäß einem auf der Leitung T liegenden Takt erfolgt. Durch das Anlegen eines Bits am Eingang E erfolgt somit aufeinander folgend für jedes Elektrodenpaar die Generierung eines Signals QO, Q1, ..., Qn, das dem Gate des Transistors des entsprechenden Elektrodenpaars zugeführt wird, wodurch das Schalten des Transistors und damit die Ausgabe des über den Eingangsverstärker V verstärkten Elektrodensignals auf den Signalbus 5 ausgelöst wird.

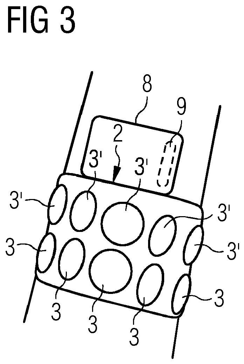

Fig. 3 zeigt die Rückseite des in Fig. 1 dargestellten Elektrodenbands 2. Man erkennt, dass an der Oberkante des Elektrodenbands ein weiteres Bauteil in der Form eines Mikrocontrollers 8 vorgesehen ist, an den die Pegel des Signalsbusses 5 weitergeleitet werden. Dabei ist ferner ein (nicht gezeigter) Analog-Digital-Wandler vorgesehen, der analoge Signale des Signalbusses in von dem Mikrocontroller verarbeitbare digitale Signale wandelt. Im Gehäuse des Mikrocontrollers ist ferner ein Vibrationsgeber 9 integriert, der einem Benutzer über taktile Ausgaben eine Rückmeldung in Bezug auf die von ihm durch seine Finger bzw. Hand ausgeführten Befehle geben kann. Insbesondere kann dabei ein Vibrationssignal ausgegeben werden, wenn eine Tastenbetätigung durch die erfindungsgemäße Bedieneinrichtung erfasst wurde.

Wie bereits oben beschrieben, erhält der Benutzer auch über einen entsprechenden Bildschirm Rückmeldung über die von ihm ausgeführten Befehle. Führt der Benutzer beispielsweise eine durch die medizinischen Vorrichtung durchzuführende Bewegung aus, kann auf dem Bildschirm ein zweidimensionales oder dreidimensionales Bild der medizinischen Vorrichtung wiedergegeben werden und die durch die Bedieneinrichtung aufgrund der Handbewegung des Benutzers ermittelte Bewegung der Vorrichtung als Simulation dargestellt werden. Entspricht die Bewegung der gewünschten Bewegung des Benutzers, kann dieser dann z.B. über eine entsprechende virtuelle Tastenbetätigung eine Betätigung eingeben, woraufhin die Bewegung durch die medizinische Vorrichtung tatsächlich ausgeführt wird. Wie ebenfalls oben erwähnt, können auch alphanumerische Eingaben des Benutzers auf dem Bildschirm bestätigt werden, falls die Eingabe von Zeichen beabsichtigt ist. Falls auch Benutzereingaben von Ereignissen gewünscht sind, die keine unmittelbare Ausgabe auf einem Bildschirm benötigen, können auch weitere Ausgabekanäle in der Vorrichtung genutzt werden, insbesondere der oben beschriebenen Vibrationsgeber bzw. auch akustische Signalgeber und dergleichen.

Die im Vorangegangenen beschriebene Ausführungsform der Erfindung weist eine Reihe von Vorteilen auf. Insbesondere kann eine Vorrichtung ohne Berührungen durch einen Benutzer bedient werden, was insbesondere bei medizinischen Vorrichtungen vorteilhaft ist, welche in steriler Umgebung eingesetzt werden. Dabei besteht die Möglichkeit, über die Bewegung der Finger bzw. der Hand in der Luft sowohl alphanumerische Eingaben als auch gegebenenfalls Bewegungsabläufe der bedienten Vorrichtung zu spezifizieren. Um solche berührungslose Eingaben zu ermöglichen, wird ein Sensor zur Erfassung der Fingerbewegung in geeigneter Weise mit einem Lagesensor zur Erfassung der Lage der Hand des Benutzers kombiniert. Dabei kann gegebenenfalls durch den Benutzer festgelegt werden, wie die Veränderungen in der Lage des Lagesensors jeweils zu interpretieren sind. Der Lagesensor, der in dem vorangegangenen Ausführungsbeispiel auf dem Handrücken vorgesehen ist, kann von dem Benutzer kontinuierlich getragen werden, so dass gewährleistet ist, dass bei der Bedienung der Vorrichtung sterile Bedingungen vorliegen. Ebenso können bestimmte Bewegungen des Lagesensors zur Feststellung von Betriebszuständen bzw. zur Erfassung diskreter Informationen interpretiert werden, z.B. in der Form eines Mausklicks. D.h., auch über den Lagesensor können analog zu dem Fingersensor gegebenenfalls entsprechende Tastenbetätigungen erfasst werden.

## Patentansprüche

1. Bedieneinrichtung für eine technische Vorrichtung, insbesondere eine medizinische Vorrichtung, wobei ein Benutzer über die Bedieneinrichtung mittels seiner Hand (H) Befehle für den Betrieb der technischen Vorrichtung generieren kann, umfassend:
- eine erste Sensoreinrichtung (1) zum Erfassen der Lage der Hand (H) des Benutzers im Raum, wodurch erste Sensorsignale (S1) generiert werden;
- eine unabhängig von der ersten Sensoreinrichtung (1) sensierende zweite Sensoreinrichtung (2) zum Erfassen der Bewegung zumindest eines Teils der Finger der Hand (H) des Benutzers, wodurch zweite Sensorsignale (S2) generiert werden;
- eine Verarbeitungseinheit (4) zum Verarbeiten der ersten und zweiten Sensorsignale (S1, S2) derart, dass ermittelt wird, welches oder welche vorbestimmten, jeweilige Befehle des Benutzers repräsentierende Bewegungsmuster die ersten und zweiten Sensorsignale (S1, S2) darstellen;
**dadurch gekennzeichnet, dass**
dieVerarbeitungseinheit (4) derart ausgestaltet ist, dass sie aus den ersten Sensorsignalen (S1) Bewegungen der Hand (H) des Benutzers ermitteln kann, welche vorbestimmte, durch die technische Vorrichtung durchzuführende Bewegungen repräsentieren, und aus den zweiten Sensorsignalen (S2) virtuelle, über zumindest einen Teil der Finger der Hand (H) des Benutzers durchgeführte Tastenbetätigungen einer virtuellen Eingabeeinrichtung ermitteln kann, und die Verarbeitungseinheit (4) derart ausgestaltet ist, dass sie im Rahmen der Ermittlung der virtuellen Tastenbetätigungen ferner bestimmen kann, welche Taste der virtuellen Eingabeeinrichtung betätigt wird, wobei hierzu neben den zweiten Sensorsignalen (S2) die ersten Sensorsignale (S1) ausgewertet werden, um über die ersten Sensorsignale (S1) die Position der Hand (H) des Benutzers in Bezug auf die virtuelle Eingabeeinrichtung während einer virtuellen Tastenbetätigung zu ermitteln.

2. Bedieneinrichtung nach Anspruch 1, wobei die virtuellen Tastenbetätigungen die Betätigung einer virtuellen Tastatur oder einer virtuellen Maus darstellen.

3. Bedieneinrichtung nach einem der vorhergehenden Ansprüche, wobei die erste Sensoreinrichtung (1) einen BeschleunigungsSensor umfasst, der insbesondere einen oder mehrere Drehratensensoren und einen oder mehrere Linearbeschleunigungs-Sensoren umfasst, wobei der oder die Drehratensensoren zur Detektion von dreidimensionalen Rotationsgeschwindigkeiten und der oder die Linearbeschleunigungs-Sensoren zur Detektion von dreidimensionalen Linearbeschleunigungen dienen.

4. Bedieneinrichtung nach Anspruch 3, wobei die erste Sensoreinrichtung (1) ferner einen Magnetfeldsensor zum Erfassen des Erdmagnetfelds umfasst.

5. Bedieneinrichtung nach Anspruch 3 oder 4, wobei die erste Sensoreinrichtung (1) auf dem Handrücken der Hand (H) des Benutzers befestigbar ist.

6. Bedieneinrichtung nach einem der vorhergehenden Ansprüche, wobei die zweite Sensoreinrichtung (2) eine elektromyographische Sensoreinrichtung zur Erfassung der elektrischen Muskelaktivität bei der Bewegung der Finger der Hand (H) des Benutzers umfasst.

7. Bedieneinrichtung nach Anspruch 6, wobei die zweite Sensoreinrichtung (2) ein am Unterarm des Benutzers befestigbares Elektrodenband umfasst, in dem eine Mehrzahl von Elektroden (3, 3') zur Erfassung der elektrischen Muskelaktivität enthalten ist.

8. Bedieneinrichtung nach Anspruch 7, wobei die Elektroden (3, 3') in dem Elektrodenband in Elektrodenpaaren von jeweils zwei sich bei Befestigung des Elektrodenbands am Unterarm in Längsrichtung des Unterarms erstreckenden Elektroden (3, 3') angeordnet sind.

9. Bedieneinrichtung nach Anspruch 8, wobei die Elektrodenpaare (3, 3') mit einem Signalbus (5) zum Übermitteln der über die jeweiligen Elektrodenpaare (3, 3') erfassten Muskelaktivität an eine Auswerteeinheit (8), insbesondere einen Mikrocontroller, verbindbar sind.

10. Bedieneinrichtung nach einem der Ansprüche 7 bis 9, wobei die Auswerteeinheit (8) am Elektrodenband angeordnet ist oder darin integriert ist.

11. Bedieneinrichtung nach Anspruch 8 oder 9 oder nach Anspruch 10 in Kombination mit Anspruch 8 oder 9, wobei für jedes Elektrodenpaar ein Eingangsverstärker (V) zur Verstärkung der vom jeweiligen Elektrodenpaar erfassten Signale vorgesehen ist.

12. Bedieneinrichtung nach Anspruch 9 oder nach Anspruch 10 oder 11 in Kombination mit Anspruch 9, wobei die von den Elektrodepaaren stammenden Signale über ein Schieberegister aufeinanderfolgend von einem Elektrodenpaar zum nächsten auf den Signalbus (5) gelegt werden.

13. Bedieneinrichtung nach einem der vorhergehenden Ansprüche, wobei eine Ausgabeeinrichtung (9) vorgesehen ist, welche derart mit der Verarbeitungseinheit (4) wechselwirkt, dass sie im Betrieb der Bedieneinrichtung dem Benutzer die von ihm generierten Befehle bestätigt.

14. Bedieneinrichtung nach Anspruch 13, wobei die Ausgabeeinrichtung eine optische Ausgabeeinrichtung, insbesondere einen Bildschirm, und/oder eine akustischen Ausgabeeinrichtung und/oder eine haptische Ausgabeeinrichtung (9) umfasst.

15. Bedieneinrichtung nach Anspruch 14, wobei die haptische Ausgabeeinrichtung (9) einen oder mehrere Vibrationsgeber umfasst, welche vorzugsweise in der ersten und/oder zweiten Sensoreinrichtung (1, 2) integriert sind.

16. Bedieneinrichtung nach einem der vorhergehenden Ansprüche, wobei die Bedieneinrichtung derart ausgestaltet ist, dass in deren Betrieb die ersten und/oder zweiten Sensorsignale (S1, S2) drahtlos, insbesondere über Bluetooth und/oder WLAN, und/oder drahtgebunden an die Verarbeitungseinheit {4) übermittelt werden.

17. Technische Vorrichtung, insbesondere medizinische Vorrichtung, umfassend eine Bedieneinrichtung nach einem der vorhergehenden Ansprüche, wobei der oder die jeweiligen Befehle gemäß den von der Bedieneinrichtung ermittelten Bewegungsmustern von der technischen Vorrichtung in deren Betrieb ausgeführt werden.

18. Technische Vorrichtung nach Anspruch 17, wobei die technische Vorrichtung eine bildgebende medizinische Vorrichtung umfasst, insbesondere eine Röntgeneinrichtung und besonders bevorzugt einen C-Bogen.

## Claims

1. Operating device for a technical apparatus, in particular a medical apparatus, wherein a user can use his hand (H) to generate commands for operating the technical apparatus via the operating device, comprising:
- a first sensor device (1) for capturing the position of the hand (H) of the user in space, as a result of which first sensor signals (S1) are generated;
- a second sensor device (2) which senses independently of the first sensor device (1) and is intended to capture the movement of at least one part of the finger of the hand (H) of the user, as a result of which second sensor signals (S2) are generated;
- a processing unit (4) for processing the first and second sensor signals (S1, S2) in such a manner that it is determined which predetermined movement pattern(s) representing respective commands from the user is/are represented by the first and second sensor signals (S1, S2);
**characterized in that**
the processing unit (4) is configured in such a manner that it can use the first sensor signals (S1) to determine movements of the hand (H) of the user which represent predetermined movements to be carried out by the technical apparatus and can use the second sensor signals (S2) to determine virtual key activations of a virtual input device which are carried out via at least one part of the finger of the hand (H) of the user, and the processing unit (4) is configured in such a manner that it can also determine, when determining the virtual key activations, which key of the virtual input device is activated, for which purpose the first sensor signals (S1) are evaluated in addition to the second sensor signals (S2) in order to use the first sensor signals (S1) to determine the position of the hand (H) of the user with respect to the virtual input device during a virtual key activation.

2. Operating device according to Claim 1, wherein the virtual key activations represent the activation of a virtual keyboard or a virtual mouse.

3. Operating device according to one of the preceding claims, wherein the first sensor device (1) comprises an acceleration sensor which comprises, in particular, one or more rotation rate sensors and one or more linear acceleration sensors, wherein the rotation rate sensor(s) is/are used to detect three-dimensional rotational speeds and the linear acceleration sensor(s) is/are used to detect three-dimensional linear accelerations.

4. Operating device according to Claim 3, wherein the first sensor device (1) also comprises a magnetic field sensor for capturing the Earth's magnetic field.

5. Operating device according to Claim 3 or 4, wherein the first sensor device (1) can be fastened to the back of the hand (H) of the user.

6. Operating device according to one of the preceding claims, wherein the second sensor device (2) comprises an electromyographical sensor device for capturing the electrical muscle activity during movement of the finger of the hand (H) of the user.

7. Operating device according to Claim 6, wherein the second sensor device (2) comprises an electrode band which can be fastened to the user's forearm and includes a plurality of electrodes (3, 3') for capturing the electrical muscle activity.

8. Operating device according to Claim 7, wherein the electrodes (3, 3') in the electrode band are arranged in electrode pairs of two electrodes (3, 3') in each case which extend in the longitudinal direction of the forearm when the electrode band is fastened to the forearm.

9. Operating device according to Claim 8, wherein the electrode pairs (3, 3') can be connected to a signal bus (5) for transmitting the muscle activity captured via the respective electrode pairs (3, 3') to an evaluation unit (8), in particular a microcontroller.

10. Operating device according to one of Claims 7 to 9, wherein the evaluation unit (8) is arranged on the electrode band or is integrated therein.

11. Operating device according to Claim 8 or 9 or according to Claim 10 in combination with Claim 8 or 9, wherein an input amplifier (V) for amplifying the signals captured by the respective electrode pair is provided for each electrode pair.

12. Operating device according to Claim 9 or according to Claim 10 or 11 in combination with Claim 9, wherein the signals coming from the electrode pairs are successively placed onto the signal bus (5) from one electrode pair to the next via a shift register.

13. Operating device according to one of the preceding claims, wherein provision is made of an output device (9) which interacts with the processing unit (4) in such a manner that it provides the user with confirmation of the commands generated by the latter during operation of the operating device.

14. Operating device according to Claim 13, wherein the output device comprises an optical output device, in particular a screen, and/or an acoustic output device and/or a haptic output device (9).

15. Operating device according to Claim 14, wherein the haptic output device (9) comprises one or more vibration transducers which are preferably integrated in the first and/or second sensor device (1, 2).

16. Operating device according to one of the preceding claims, wherein the operating device is configured in such a manner that the first and/or second sensor signals (S1, S2) are transmitted wirelessly, in particular via Bluetooth and/or WLAN, and/or in a wired manner to the processing unit (4) during operation of the operating device.

17. Technical apparatus, in particular medical apparatus, comprising an operating device according to one of the preceding claims, wherein the respective command(s) is/are carried out by the technical apparatus during its operation according to the movement patterns determined by the operating device.

18. Technical apparatus according to Claim 17, wherein the technical apparatus comprises an imaging medical apparatus, in particular an x-ray device and particularly preferably a C-arm.

## Revendications

1. Système de commande pour un dispositif technique, notamment un dispositif médical, ledit système de commande permettant à un utilisateur de générer au moyen de sa main (H) des commandes concernant le fonctionnement du dit dispositif technique, comprenant
- un premier moyen de capteur (1) destiné à détecter la position de la main (H) de l'utilisateur dans l'espace, et générant des premiers signaux (S1) de capteur,
- un deuxième moyen de capteur (2) dont la détection est indépendante du premier moyen de capteur (1) et destiné à détecter le mouvement d'au moins une partie des doigts de la main (H) de l'utilisateur, en générant ainsi des deuxièmes signaux (S2) de capteur, et
- une unité de traitement (4) destinée à traiter les premiers et deuxièmes signaux de capteur (S1, S2) de façon à déterminer lequel ou lesquels schémas de mouvement prédéfinis, qui sont chacun représentatif d'une commande de l'utilisateur, est(sont) représenté(s) par les premiers et deuxièmes signaux de capteur (S1, S2),
**caractérisé en ce que**
l'unité de traitement (4) est conçue de façon à pouvoir déterminer, à partir des premiers signaux de capteur (S1) des mouvements de la main (H) de l'utilisateur qui représentent des mouvements prédéterminés à exécuter par le dispositif technique et à pouvoir déterminer, à partir des deuxièmes signaux de capteur (S2), des actionnements virtuels de touche d'un moyen d'entrée virtuel exécutés par au moins une partie des doigts de la main (H) de l'utilisateur, et l'unité de traitement (4) est conçue de façon à pouvoir déterminer en outre, dans le cadre de la détermination des actionnements virtuels de touche, quelle touche du moyen virtuel d'entrée est actionnée, en analysant pour cela les premiers signaux de capteur (S1) en plus des deuxièmes signaux de capteur (S2) afin de déterminer, par les premiers signaux de capteur (S1), la position de la main (H) de l'utilisateur par rapport au moyen d'entrée virtuel pendant un actionnement de touche virtuel.

2. Système de commande selon la revendication 1, dans lequel les actionnements virtuels de touche représentent un actionnement d'un clavier virtuel ou d'une souris virtuelle.

3. Système de commande selon l'une quelconque des revendications précédentes, dans lequel le premier moyen de capteur (1) comprend un capteur d'accélération, qui comporte notamment un ou plusieurs capteurs de vitesse angulaire et un ou plusieurs capteurs d'accélération linéaire, le ou les capteurs de vitesse angulaire servant à détecter les vitesses angulaires en trois dimensions et le ou les capteurs d'accélération linéaire servant à détecter les accélérations linéaires en trois dimensions.

4. Système de commande selon la revendication 3, dans lequel le premier moyen de capteur (1) comprend en outre un capteur de champ magnétique destiné à détecter le champ magnétique terrestre.

5. Système de commande selon la revendication 3 ou 4, dans lequel le premier moyen de capteur (1) peut être fixé sur le dos de la main (H) de l'utilisateur.

6. Système de commande selon l'une quelconque des revendications précédentes, dans lequel le second moyen de capteur (2) comprend un moyen de capteur électro-myographique destiné à détecter l'activité musculaire électrique lors du mouvement des doigts de la main (H) de l'utilisateur.

7. Système de commande selon la revendication 6, dans lequel le second moyen de capteur (2) comprend une bande d'électrodes pouvant être fixée à l'avant-bras de l'utilisateur et qui contient une pluralité d'électrodes (3, 3') destinées à détecter l'activité musculaire électrique.

8. Système de commande selon la revendication 7, dans lequel les électrodes (3, 3') dans la bande d'électrodes sont disposées en paires d'électrodes comportant chacune deux électrodes (3, 3') s'étendant dans le sens de la longueur de l'avant-bras lors de la fixation de la bande d'électrodes sur l'avant-bras.

9. Système de commande selon la revendication 8, dans lequel les paires d'électrodes (3, 3') peuvent être connectées à un bus de signal (5) permettant de transmettre l'activité musculaire détectée par la paire d'électrodes respective (3, 3') à une unité d'évaluation (8), notamment à un microcontrôleur.

10. Système de commande selon l'une quelconque des revendications 7 à 9, dans lequel l'unité d'évaluation (8) est disposée sur la bande d'électrode ou est intégrée à l'intérieur de celle-ci.

11. Système de commande selon la revendication 8 ou 9 ou la revendication 10 combinée à la revendication 8 ou 9, dans lequel, pour chaque paire d'électrodes, un amplificateur d'entrée (V) destiné à amplifier les signaux détectés par la paire d'électrodes respective est prévu.

12. Système de commande selon la revendication 9 ou la revendication 10 ou 11 combinée à la revendication 9, dans lequel les signaux provenant des paires d'électrodes sont appliqués successivement d'une paire d'électrodes à l'autre au bus de signal (5) via un registre à décalage.

13. Système de commande selon l'une quelconque des revendications précédentes, dans lequel un dispositif de sortie est prévu, lequel interagit avec l'unité de traitement (4) de telle sorte qu'il confirme à l'utilisateur les commandes générées par celui-ci durant le fonctionnement du système de commande.

14. Système de commande selon la revendication 13, dans lequel le dispositif de sortie comprend un dispositif de sortie optique, notamment un écran, et/ou un dispositif de sortie acoustique et/ou un dispositif de sortie haptique (9).

15. Système de commande selon la revendication 13, dans lequel le dispositif de sortie haptique (9) comprend un ou plusieurs vibreur(s) qui est(sont) intégré(s) de préférence dans le premier et/ou deuxième moyen de capteur (1, 2).

16. Système de commande selon l'une quelconque des revendications précédentes, le système de commande étant conçu de telle sorte que, lors de son fonctionnement, les premiers et/ou deuxièmes signaux de capteur (S1, S2) sont transmis à l'unité de traitement (4) sans fil, notamment par Bluetooth et/ou par réseau local sans fil (WLAN) et/ou par liaison filaire.

17. Dispositif technique, notamment dispositif médical, comprenant un système de commande selon l'une quelconque des revendications précédentes, dans lequel la ou les commandes respectives sont exécutées par le dispositif technique lors de son fonctionnement selon les schémas de mouvement déterminés par le système de commande.

18. Dispositif technique selon la revendication 17, qui comprend un dispositif médical d'imagerie, notamment un dispositif à rayons X et de préférence un bras en C.
